# EUROPEAN PATENT SPECIFICATION

(11) **EP 1 243 246 B1**
(45) Date of publication and mention of the grant of the patent: **17.11.2004**
(21) Application number: 01106787.3
(22) Date of filing: 19.03.2001
(51) Int. Cl.: A61K 6/10

(54) **Composition for dental impression material**
Zahnabdruckmaterial
Composition pour la prise d'empreintes dentaires

(43) Date of publication of application: 25.09.2002
(73) Proprietor: Cavex Holland BV, NL-2031 WH Haarlem (NL)
(72) Inventor: Timmerman, Gerrit, 2013 BP Haarlem (NL); Ippel, Dirk, 2015 KL Haarlem (NL)
(74) Representative: Kühn, Hans-Christian

(56) References cited:
- EP-A- 0 086 062
- AU-B- 461 693
- GB-A- 776 501
- GB-A- 1 044 160
- US-A- 2 816 843

## Description

The invention relates to a composition for a dental impression material, use of a mixture and a dental impression material.

Alginate impression materials are being used for many years in dentistry. They are used to make impressions for prosthodontic, orthodontic or other appliances.

In general an alginate impression material consists of a powder, which is mixed with water to form a paste. This paste is filled into an impression tray, which is placed in the mouth of the patient. The paste forms a rubber-like gel in a few minutes time. This negative impression is poured with dental stone. The resulting gypsum model is an exact copy of the situation in the mouth and can be used to form and produce the desired prosthetic device.

The setting of an alginate impression material is based on the reaction between alginate, a long-chain polysaccharide, and calcium ions. The usual calcium source is the slightly soluble calcium sulphate. In order to give the dentist sufficient time to fill the impression tray and to take the impression, the setting reaction must be suppressed for some time. For this purpose sequestering agents like sodium phosphate and/or sodium silicate are added. These components react with the released calcium ions, thereby postponing the setting reaction until they are completely depleted.

Alginate impression materials further contain metal oxides and fluorides to control the pH and therewith the compatibility with gypsum and the detail reproduction. Also fillers like diatomaceous earth are added to facilitate the water take up and to reinforce the final gel.

The timing for mixing and using alginate impression material is fairly critical. The time that powder and water are mixed must be long enough to enable the reactive components in the material to go into solution. On the one hand a fast setting is desirable to cause as little discomfort as possible to the patient, on the other hand if the setting reaction has progressed too far at the moment that the impression is taken, the flow properties of the material are insufficient to give an exact reproduction of the situation in the mouth.

Materials that indicate the stage of the setting by means of the colour are well known (e. g. AU 461 693 ). The initial pH of alginate impression materials is high, through the presence of basic components like sodium phosphate and sodium silicate. When the concentration of these components decreases, due to the reaction with calcium, the pH will also drop. By adding pH depending dyes, the progression of the setting reaction can be followed visually. Common colorants for this purpose are thymolphthalein, changing from blue to colourless at a pH 10 - 9, and phenolphthalein changing from pink to colourless at pH 9 - 8. The usual colour changes in impression materials are violet to pink at the end of the mixing time and pink to white at the end of the working time.

The colour change at the end of the working time, as shown by many materials currently on the market, is of little importance. Usually the dentist will place the impression tray in the mouth, as soon as the material is mixed and filled into the tray. The transition that is of more interest for both dentist and patient, the moment that the impression has set and can be taken out of the mouth, is not visible.

GB 1044160 describes a method to improve the quality of the gypsum model that is obtained from an alginate impression. A pH drop during the setting process is visualized by indicator colorants. However, there is no direct link between the setting reaction and the colour change.

The problem in this respect is to avoid some of the above mentioned disadvantages. The main issue is to provide a composition for a dental impression material which has three clear time phased colour changes, showing both mixing and working and setting times.

This problem is solved by a composition according to claim 1, a use of a mixture according to claim 3 and a dental impression material according to claim 5.

The composition for dental impression material comprises a sodium/or potassium alginate, calcium sulphate-dihydrate and/or calciumsulphate-hemihydrate, magnesium oxide, sodium and/or potassium silicate, tetrasodium and/or potassium pyrophosphate, diatomaceous earth, thymolphthalein and phenolphthalein.

The composition is characterized by:
- alginate (K- and/or Na-): 10 - 15 % (m/m),
- calciumsulphate-hydrate-di- and/or -hemihydrate): 3 - 10 % (m/m),
- magnesium oxide: 1 - 5 % (m/m),
- potassium and/or sodium fluoro titanate: 1 - 4 % (m/m),
- tetra sodium and/or potassium pyrophosphate: 0,1 - 1 % (m/m),
- phenolphthalein: 0,01 % (m/m),
- mixture A: 3 % (m/m), as described in claim 1,
- magnesium silicate: 0 - 15 % (m/m),
- diatomaceous earth: 82 - 53 % (m/m).

The present invention is related to a material that is showing an extra colour change and thereby also giving information about the progression of the setting of the impression. The colour of the invented material is blue during the mixing time, violet during the working time, pink during the setting time and white when the impression has set.

The blue colour during mixing is achieved by blending the colour indicator concerned (thymolphthalein) with one of the sequestering agents, before it is put into the final formulation. Due to the basic nature of these agents, the colorant is brought in the coloured state beforehand. When such a product is mixed with water the blue colour will be visible right from the start whereas the pink colour from the phenolphthalein will follow later.

According to the invention it is surprisingly found that it is necessary to use a type of calcium sulphate in which the particles have a clear defined crystalline structure. This will limit the initial release of calcium ions. In combination with an optimized percentage of calcium sulphate in the formulation, this will enable the basic components to cause a sufficiently high pH to maintain the blue colour during the mixing time. Due to this delayed pH drop it is also accomplished that the colour change of the thymolphthalein is at the end of the working time and the colour change of the phenolphthalein at the end of the setting time. The usual time schedule for the handling of alginate impression material is a mixing time of 30 to 40 s, a working time of 30 to 40 s and a setting time of 60 s. Both working and setting times are influenced by factors like water temperature, water hardness and ambient temperature. Because the colour changes are pH dependent and not time dependent, the colour of the product as described will indicate the progression of the setting under all conditions.

In our opinion there are two reasons that the invented composition has clear three time phase colour changes. The first is that the thymolphthalein is premixed with the base (see mixture A) and is already coloured when it comes in contact with water. The second is the calcium concentration which is responsible for the pH drop is controlled by selecting the right type of calcium source (di- and/or hemihydrate) and by using the right percentage in the formulation.

It is a preferred embodiment that the invented composition is characterized by:
- diatomaceous earth: 70 % (m/m),
- alginate (K- and/or Na-): 12 % (m/m),
- calciumsulphate (di- and/or hemihydrate): 6 % (m/m),
- magnesium silicate: 5 % (m/m),
- magnesium oxide: 2 % (m/m),
- potassium and/or fluoro titanate: 2 % (m/m),
- tetra sodium and/or potassium pyrophosphate: 0,4 % (m/m),
- phenolphthalein: 0,01 % (m/m),
- mixture A: 3 % (m/m),
whereby the mixture A is comprising of:
- thymolphthalein: 0,8 % (m/m),
- sodium and/or potassium silicate: 11 % (m/m),
- diatomaceous earth: 88 % (m/m).

A further object of the invention is the use of a mixture comprising of thymolphthalein, sodium and/or potassium silicate and diatomaceous earth as an ingredient of a composition for a dental impression material as described in claim 1 on account of the above mentioned reasons.

On account of practical superior characteristics the use of such a mixture is preferred which is characterized by
- thymolphtalein: 0,8 % (m/m),
- sodium and/or potassium silicate: 11 % (m/m),
- diatomaceous earth: 88 % (m/m).

Finally a dental impression material which comprises a composition according to the invention shows the above superior characteristics.

The invention is described by the following examples.

In the examples given below, the components in powder form are blended in a suitable powder mixer until homogeneous. If one or more of the ingredients contain particles exceeding 150 µm, these particles are to be removed or reduced in size by means of a sieving or milling process.

For the evaluation of the products, 22 g of the powder is mixed with 50 ml of demineralised water of 23 °C with a spatula in a mixing bowl. The changes in colour are assessed visually and the working time is determined according to the method described in ANSI/ADA specification no. 18, i.e. the moment that the material does not adhere anymore to a PMMA rod. The hardening time is the working time plus one minute.

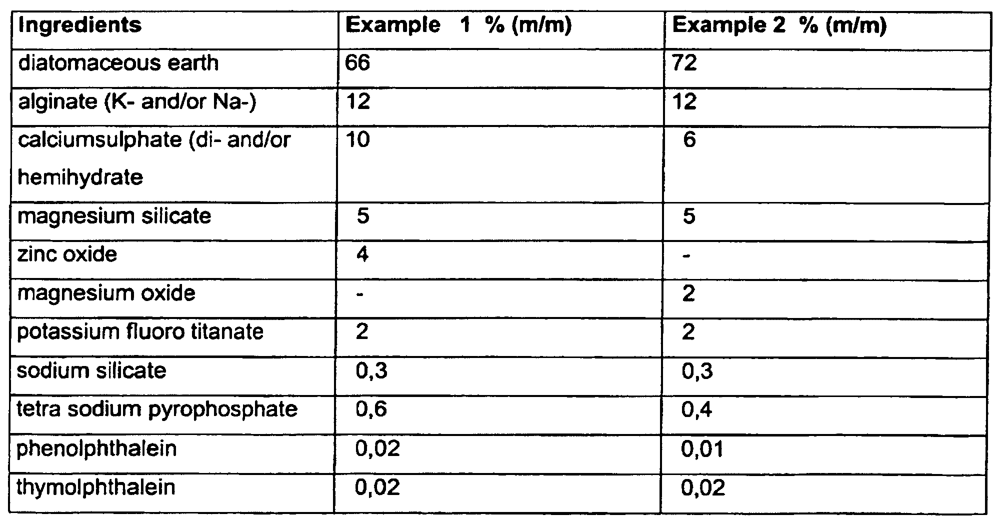

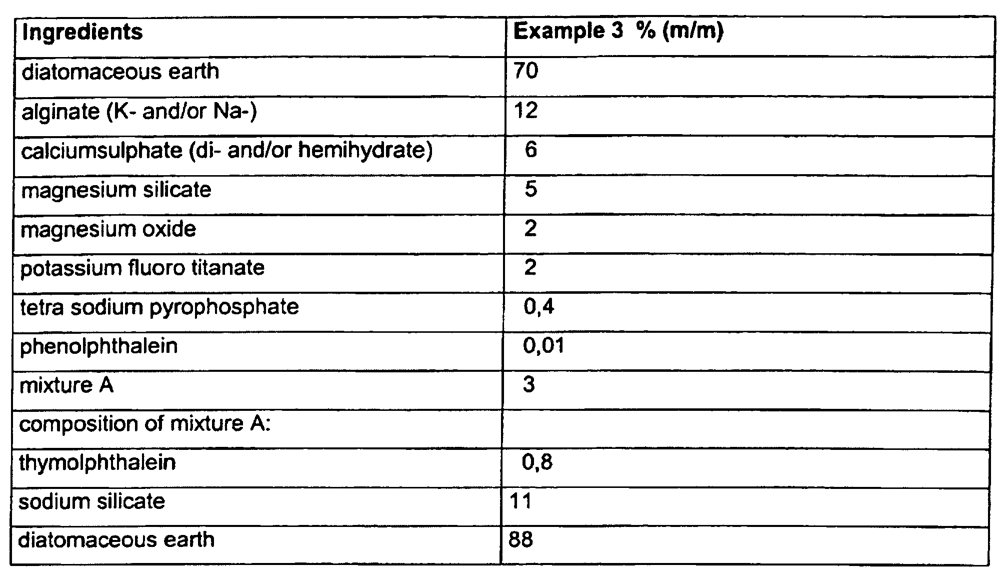

Mixture A is prepared by blending intimately together the base and the colour indicator with diatomaceous earth as filler in a powder blender or ball mill. The filler is added to facilitate the mixing process. The water present in the components as crystal water or physically bound water is sufficient to act as a solvent in which the colorant can change to the blue colour.

In example 1 the material has a violet colour for a short time (10 - 20 s) turning to pink until the end of the setting time. The colour change at the end of the mixing time is not clear.

In example 2 the material is violet during 30 - 40 s and pink afterwards until the end of the working time. Compared to example 1 the colour change can be distinguished much clearer.

Example 3 is the present invention. Unlike the powders of 1 and 2, that have a white colour, the powder of example 3 has a light blue to violet shade. When the powder comes in contact with water, it gets a clear blue colour immediately, which remains visible during the mixing time of 30 - 40 s and is followed by a violet colour for 30 - 40 s during the working time, pink during the next minute and finally white at the end of the setting.

## Claims

1. Composition for a dental impression material comprising a sodium and/or potassium alginate, calcium sulphate-dihydrate and/or calciumsulphate-hemihydrate, magnesium oxide, sodium and/or potassium silicate, tetrasodium and/or potassium pyrophosphate, diatomaceous earth, thymolphthalein and phenolphthalein, **characterized by**:
- sodium and/or potassium alginate: 10 - 15 % (m/m),
- calciumsulphate-hydrate-di- and/or -hemihydrate): 3 - 10 % (m/m),
- magnesium oxide: 1 - 5 % (m/m),
- potassium and/or sodium fluoro titanate: 1 - 4 % (m/m),
- tetra sodium and/or potassium pyrophosphate: 0,1 - 1 % (m/m),
- phenolphthalein: 0,01 %(m/m),
- mixture A: 3 % (m/m),
- magnesium silicate: 0 - 15 % (m/m),
- diatomaceous earth: 82 - 53 % (m/m),
whereby the mixture A is comprising of:
- thymolphthalein: 0,8 % (m/m),
- sodium and/or potassium silicate: 11 % (m/m),
- diatomaceous earth: 88 % (m/m).

2. Composition according to claim 1, **characterized by**:
- diatomaceous earth: 70 % (m/m),
- alginate (K- and/or Na-): 12 % (m/m),
- calciumsulphate (di- and/or hemihydrate): 6 % (m/m),
- magnesium silicate: 5 % (m/m),
- magnesium oxide: 2 % (m/m),
- potassium and/or sodium fluoro titanate: 2 % (m/m),
- tetra sodium and/or potassium pyrophosphate: 0,4 % (m/m),
- phenolphthalein: 0,01 % (m/m),
- mixture A: 3 % (m/m),
whereby the mixture A is comprising of:
- thymolphthalein: 0,8 % (m/m),
- sodium and/or potassium silicate: 11 % (m/m),
- diatomaceous earth: 88 % (m/m).

3. Use of a mixture comprising of thymolphthalein, sodium and/or potassium silicate and diatomaceous earth as an ingredient of a composition for a dental impression material according to claim 1.

4. Use according to claim 3, **characterized by** a mixture comprising of:
- thymolphthalein: 0,8 % (m/m),
- sodium and/or potassium silicate: 11 % (m/m),
- diatomaceous earth: 88 % (m/m).

5. Dental impression material, comprising a composition according to one of the claims 1 to 2.

## Patentansprüche

1. Zusammensetzung für ein Zahnabformmaterial bestehend aus einem Natriumund/oder Kaliumalginat, Kalziumsulfat-Dihydrat und/oder Kalziumsulfat-Hemihydrat, Magnesiumoxid, Natrium- und/oder Kaliumsilikat, Tetranatrium- und/oder - kaliumpyrophosphat, Diatomeenerde, Thymolphthalein und Phenolphthalein, **gekennzeichnet durch**:
- Natrium- und/oder Kaliumalginat: 10-15 Gew-%,
- Kalziumsulfat-Hydrat (-Dihydrat und/oder -Hemihydrat): 3 - 10 Gew-%,
- Magnesiumoxid: 1 - 5 Gew-%,
- Kalium- und/oder Natriumfluortitanat: 1 - 4 Gew-%,
- Tetranatrium- und/oder -kaliumpyrophosphat: 0,1 - 1 Gew-%,
- Phenolphthalein: 0,01 Gew-%,
- Mischung A: 3 Gew-%,
- Magnesiumsilikat: 0 - 15 Gew-%,
- Diatomeenerde: 82 - 53 Gew-%,
wobei die Mischung A besteht aus:
- Thymolphthalein: 0,8 Gew-%,
- Natrium- und/oder Kaliumsilikat: 11 Gew-%,
- Diatomeenerde: 88 Gew-%.

2. Zusammensetzung nach Anspruch 1, **gekennzeichnet durch**:
- Diatomeenerde: 70 Gew-%,
- Alginat (K- und/oder Na-): 12 Gew-%,
- Kalziumsulfat (Di- und/oder Hemihydrat): 6 Gew-%,
- Magnesiumsilikat: 5 Gew-%,
- Magnesiumoxid: 2 Gew-%,
- Kalium- und/oder Natriumfluortitanat: 2 Gew-%,
- Tetranatrium- und/oder -kaliumpyrophosphat: 0,4 Gew-%,
- Phenolphthalein: 0.01 Gew-%,
- Mischung A: 3 Gew-%,
wobei die Mischung A besteht aus:
- Thymolphthalein: 0,8 Gew-%,
- Natrium- und/oder Kaliumsilikat: 11 Gew-%,
- Diatomeenerde: 88 Gew-%.

3. Verwendung einer Mischung bestehend aus Thymolphthalein, Natrium- und/oder Kaliumsilikat und Diatomeenerde als ein Bestandteil einer Zusammensetzung für ein Zahnabformmaterial nach Anspruch 1.

4. Verwendung nach Anspruch 3, **gekennzeichnet durch** eine Mischung bestehend aus:
- Thymolphthalein: 0,8 Gew-%,
- Natrium- und/oder Kaliumsilikat: 11 Gew-%,
- Diatomeenerde: 88 Gew-%.

5. Zahnabformmaterial bestehend aus einer Zusammensetzung nach einem der Ansprüche 1 bis 2.

## Revendications

1. Composition pour une prise d'empreintes dentaires comprenant un alginate de sodium et/ou de potassium, un sulfate de calcium dihydraté et/ou un sulfate de calcium hémihydraté, un oxyde de magnésium, un silicate de sodium et/ou de potassium, un pyrophosphate de tétrasodium et/ou de potassium, une terre de diatomée, une thymolphtaléine et une phénolphtaléine, **caractérisée par** :
- un alginate de sodium et/ou de potassium : 10 à 15 % (m/m)
- un sulfate de calcium hydraté, di et/ou hémihydraté : 3 à 10 % (m/m)
- un oxyde de magnésium : 1 à 5 % (m/m)
- un fluorotitanate de potassium et/ou de sodium : 1 à 4 % (m/m)
- un pyrophosphate de tétrasodium et/ou de potassium : 0,1 à 1 % (m/m)
- une phénolphtaléine : 0,01 % (m/m)
- un mélange A : 3 % (m/m)
- un silicate de magnésium : 0 à 15 % (m/m)
- une terre de diatomée : 83 à 54 % (m/m)
le mélange A se composant de :
- thymolphtaléine : 0,8 % (m/m)
- silicate de sodium et/ou de potassium : 11 % (m/m)
- terre de diatomée : 88 % (m/m).

2. Composition selon la revendication 1, **caractérisée par** :
- une terre de diatomée : 70 % (m/m)
- un alginate (K et/ou Na) : 12 % (m/m)
- un sulfate de calcium (di et/ou hémihydraté) : 6 % (m/m)
- un silicate de magnésium : 5 % (m/m)
- un oxyde de magnésium : 2 % (m/m)
- un fluorotitanate de potassium et/ou de sodium : 2 % (m/m)
- un pyrophosphate de tétrasodium et/ou de potassium : 0,4 % (m/m)
- une phénolphtaléine : 0,01 % (m/m)
- un mélange A : 3 % (m/m)
le mélange A se composant de :
- thymolphtaléine : 0,8 % (m/m)
- silicate de sodium et/ou de potassium : 11 % (m/m)
- terre de diatomée : 88 % (m/m).

3. Utilisation d'un mélange se composant de thymolphtaléine, de silicate de sodium et/ou de potassium et de terre de diatomée en tant qu'ingrédient d'une composition pour une prise d'empreintes dentaires selon la revendication 1.

4. Utilisation selon la revendication 3, **caractérisée par** un mélange se composant de :
- thymolphtaléine : 0,8 % (m/m)
- silicate de sodium et/ou de potassium : 11 % (m/m)
- terre de diatomée : 88 % (m/m).

5. Prise d'empreintes dentaires, comprenant une composition selon l'une quelconque des revendications 1 à 2.
